# EUROPEAN PATENT APPLICATION

(11) **EP 4 517 359 A1**
(43) Date of publication of application: **05.03.2025**
(21) Application number: 23275129.7
(22) Date of filing: 30.08.2023
(51) Int. Cl.: G01R 33/421

(54) **SHIELDING ARRANGEMENT AND MAGNETIC RESONANCE DEVICE**

(71) Applicant: Siemens Healthcare Limited, Camberley, Surrey GU15 3YL (GB)
(72) Inventor: Aley, Nicholas, Maldon / Essex, CM96NF (GB); Pinho Meneses, Bruno, Witney, OX28 6AQ (GB); Ströhlein, Christopher, 90480 Nürnberg (DE)
(74) Representative: Maier, Daniel Oliver

(57) **Abstract**

The invention relates to a shielding arrangement (30) for a magnetic resonance device (11), comprising a main magnet (17) including at least one solenoid coil, a shield structure (33) formed as a double-walled hollow cylinder comprising an outer wall and an inner wall, and a shield tube (34) enclosed between the at least one solenoid coil of the main magnet (17) and the inner wall of the shield structure (33), wherein the main magnet (17) is arranged between the outer wall and the inner wall of the shield structure (33) and wherein the shield tube (34) is mechanically supported by the shield structure (33). The invention also relates to a magnetic resonance device (11) comprising an inventive shielding arrangement (30).

## Description

*Independent of the grammatical term usage, individuals with male, female or other gender identities are included within the term.*

Magnetic resonance devices typically use gradient systems for spatial encoding of magnetic resonance signals acquired during a magnetic resonance measurement. A gradient system usually comprises a plurality of gradient coils configured to generate fast-changing gradient magnetic fields in an imaging volume which is encompassed by the gradient coils. Inevitably, the gradient coils also generate fast-changing stray fields outside of the gradient coils. These stray fields can interact with a main magnet of the magnetic resonance device which usually is arranged around the gradient coil.

Shield structures enclosing the main magnet, such as cryogen vessels or thermal shields, are usually made from an electrically conductive metal (e. g. aluminium or stainless steel). When the fast-changing stray fields from the gradient coils pass through a conductive material, eddy currents are generated. These eddy currents produce ohmic heating on conducting surfaces of the shield structures and increase a demand of cooling provided via a cryogenic refrigeration system. Although the eddy currents shield the main magnet from the fast-changing stray fields of the gradient coils, magneto-mechanical interactions of the eddy currents with conducting surfaces of the cryogen vessel produce secondary magnetic fields, leading to high levels of heating in the main magnet and other low temperature surfaces of the magnetic resonance device.

Conventional magnetic resonance devices comprise cryocoolers having two distinct cooling stages: a first cooling stage at approximately 50 K for cooling the thermal shield and a second cooling stage at approximately 4 K for cooling a cryogen contained within the cryogen vessel and/or maintaining the main magnet at a superconducting temperature. The second cooling stage of the cryocooler typically provides a very low cooling capacity, e. g. between 1 W and 2 W. An increased heat load at the second cooling stage can be critical, as it may be difficult to maintain the superconducting temperature. Even small deviations from the superconducting temperature can cause a magnet quench, particularly in "dry" systems, which do not comprise a substantial amount of cryogen acting as a thermal buffer. In "wet" systems, on the other hand, increased heat loads may lead to high levels of cryogen boil-off, which is also undesirable.

Therefore, heat loads associated with eddy currents may limit an allowable gradient performance. Furthermore, eddy currents on conducting surfaces may also generate magnetic fields (i. e. secondary magnetic fields) in the imaging volume of the magnetic resonance device. These secondary magnetic fields can disturb a homogeneity of a main magnetic field and compromise the quality of acquired magnetic resonance images.

It is an object of the present invention to reduce heat loads on cryogenically cooled components of a magnetic resonance device without compromising image quality.

This objective is achieved by a shielding arrangement and a magnetic resonance device according to the invention. Further advantageous embodiments are specified in the dependent claims.

The inventive shielding arrangement for a magnetic resonance device comprises a main magnet including at least one solenoid coil, a shield structure formed as a double-walled hollow cylinder comprising an outer wall and an inner wall, and a shield tube enclosed between the at least one solenoid coil of the main magnet and the inner wall of the shield structure.

The main magnet may comprise one or more superconducting magnets. In a preferred embodiment, the main magnet comprises one or more solenoidal or cylindrical superconducting coils. The one or more solenoidal or cylindrical superconducting coils may be rotationally symmetric or comprise rotationally symmetric bodies. According to an embodiment, each solenoidal or cylindrical superconducting coil comprises an axis of rotational symmetry arranged on an axis of rotational symmetry of the main magnet. The main magnet may comprise a dedicated support structure configured to carry the main magnet and/or provide support to the main magnet. The term "main magnet" as used herein may comprise one or more solenoidal superconducting coil as well as a dedicated support structure.

The shield structure may be configured to shield the main magnet from stray fields generated by a gradient system. The shield structure may also be configured to reduce a transport of heat energy to the main magnet. A transport of heat energy may be characterized by a heat transport mechanism such as thermal radiation, heat conduction, but also heat convection. The shield structure, particularly an outer wall of the shield structure, may circumferentially encompass the main magnet.

The shield structure may form a vessel enclosing the main magnet and the shield tube. In a preferred embodiment, the thermal shield comprises an outer wall, an inner wall and annular end walls connecting the outer wall and the inner wall. Particularly, the thermal shield may form a double-walled hollow cylinder enclosing the main magnet and the shield tube between the outer wall, the inner wall and the annular end walls. The shield structure may comprise an axis of rotational symmetry oriented in parallel to or corresponding to an axis of rotational symmetry defined by the at least one solenoid coil of the main magnet.

Preferably, the shield structure comprises or consists of a material with a high electrical conductivity and/or a high thermal conductivity. For example, the shield structure may consist of copper or aluminium, particularly high purity aluminium. The shield structure may also comprise gold, platinum, silver, or other metals with a high electrical conductivity. In one embodiment, the shield structure is coated or galvanized with a material having a high electrical conductivity and/or high thermal conductivity.

The shield structure may be configured to shield the main magnet from an (electro-)magnetic field, particularly an electromagnetic field generated by a gradient system. For example, the shield structure may be configured to allow for eddy currents or screening currents to be generated along an electrically conducting surface of the shield structure in dependence of the electromagnetic field. Particularly, the shield structure may comprise an electrically conductive layer allowing for a generation of eddy currents in dependence of a primary electromagnetic field, e. g. a gradient magnetic field, particularly a stray field of a gradient magnetic field. A generated eddy current may generate a secondary (electro-)magnetic field opposing the first electromagnetic field. Particularly, the first electromagnetic field and the secondary electromagnetic field may cancel each other out to some extent, thus providing a shielding effect.

The outer wall and the inner wall of the shield structure may each comprise a continuous or coherent surface. In an alternative embodiment, the outer wall and/or inner wall of the shield structure may comprise or consist of a plurality of spaced shield elements or rings. The shield structure may further comprise one or more sections of coiled wire.

In a preferred embodiment, the shield structure is shaped in such a way to conform to a shape of the main magnet. For example, a surface contour of the shield structure may imitate or conform to a surface contour of the main magnet. According to an embodiment, the inventive shielding arrangement comprises an outer vacuum chamber. The outer vacuum chamber may represent a vessel which is substantially impermeable to fluids, such as a liquid or a gaseous cryogen. The outer vacuum chamber may enclose other components of the shielding arrangement, such as a cryogen vessel, the shield structure, the shield tube, the main magnet, and the like.

According to an embodiment, the outer vacuum chamber comprises an outer shell, an inner shell and annular end walls connecting the outer shell and the inner shell. Particularly, the outer vacuum chamber may form a double-walled hollow cylinder enclosing the main magnet and the shield structure between the outer shell, the inner shell and the annular end walls. The outer vacuum chamber may comprise an axis of rotational symmetry oriented in parallel to or corresponding to an axis of rotational symmetry defined by the at least one solenoid coil of the main magnet. The inner shell of the outer vacuum chamber may correspond to a patient bore of a magnetic resonance device.

In a preferred embodiment, the outer vacuum chamber is configured to provide mechanical support to the shield structure and/or the main magnet. For example, the main magnet and/or the shield structure may be suspended from or carried by the outer vacuum chamber.

The shield structure may be configured to block thermal radiation from a direction of the outer shell, the inner shell, and the annular end walls of the outer vacuum chamber.

The shield tube may comprise a tubular or cylindrical shape. For example, the shield tube may comprise the shape of a hollow cylinder. Preferably, the shield tube does not form a vessel or container enclosing the main magnet.

The shield tube may comprise or consist of a material having an intermediate electrical conductivity at a temperature of 300 K. According to an embodiment, the shield tube comprises or consists of a material having a thermal conductivity of less than 120 *W*/(*m·K*)*,* less than 100 *W*/(*m·K*)*,* or less than 80 *W*/(*m·K*) at a temperature of 300 K. For example, the shield tube may consist of steel or stainless steel. However, the shield tube may comprise or consist of other materials, particularly metals having a high electrical conductivity, such as copper or aluminium.

In providing a shield tube comprising or consisting of a material having an intermediate electrical conductivity, manufacturing costs of the shielding arrangement may favourably be reduced. Furthermore, an occurrence of long time-constant eddy currents, which can negatively impact image quality, may favourably be avoided.

In a preferred embodiment, the shield tube is arranged between the main magnet and the inner wall of the shield structure. However, the shield tube may also be arranged between an inner wall of a cryogen vessel and the inner wall of the shield structure.

According to the invention, the main magnet is arranged between the outer wall and the inner wall of the shield structure. For example, the main magnet may be enclosed in a vessel formed by the outer wall, the inner wall as well as the annular end walls of the shield structure. However, the main magnet may also be enclosed in a cryogen vessel which is enclosed between the outer wall and the inner wall of the shield structure.

The shield tube is mechanically supported by the shield structure. Preferably, the shield tube is mechanically supported by the inner wall of the shield structure.

According to an embodiment, the inventive shielding arrangement comprises at least one spacer mechanically connected to the shield structure and the shield tube. The at least one spacer is configured to maintain a predefined distance between the inner wall of the shield structure and the shield tube.

The at least one spacer may be arranged between the inner wall of the shield structure and the shield tube. Preferably, the at least one spacer is configured to fix the shield tube in a predefined relative position to the shield structure.

The at least one spacer may circumferentially encompass the inner wall or inner tube of the shield structure at least along a section of a circumference of the inner tube or inner wall. For example, the at least one spacer may be implemented as a ring, a plurality of rings, a hollow cylinder, a plurality of hollow cylinders, or a segment of a ring or a hollow cylinder.

According to an embodiment, a radially outer surface of the at least one spacer is connected to the shield tube, whereas a radially inner surface of the at least one spacer is connected to the inner tube or inner wall of the shield structure.

The at least one spacer may be configured to provide a force-locking mechanical connection between the shield tube and the shield structure. For example, a radial dimension of the at least one spacer may be oversized in relation to an inner diameter of the shield tube. Thus, the at least one spacer may exert a force against the shield structure and the shield tube, fixing the shield tube in a predefined relative position to the shield structure. However, the at least one spacer may also comprise a recess or cut-out configured to accommodate a section of the shield tube and provide a form-locking connection with the shield tube. It is also conceivable, that the at least one spacer is materially bonded to the shield tube and/or the shield structure via an adhesive, preferably a thermally conductive adhesive. However, the shield structure, the at least one spacer and the shield tube may be mechanically connected via any suitable form-locking, force-locking and/or material connection.

In a preferred embodiment, the at least one spacer comprises or consists of a thermally conductive material. Particularly, the at least one spacer may be configured to transport thermal energy between the shield tube and the shield structure via thermal conduction.

The at least one spacer arranged between the shield tube and the shield structure may favourably allow for a tuning of a mechanical resonance behaviour or a mechanical resonance profile of the shielding arrangement, particularly the shield structure. For example, the at least one spacer may be configured to improve or attenuate a transfer of mechanical energy between the shield tube and the shield structure. Thus, a resonance frequency of the shield structure can favourably be adjusted as required, independent of a design of the shield structure.

Furthermore, the at least one spacer may favourably allow for a tuning of an exchange of thermal energy between the shield tube and the shield structure. Particularly, the at least one spacer may be configured to provide a thermal and a mechanical connection between the shield tube and the shield structure. Thus, additional components for providing a mechanical support of the shield tube and/or a thermal contact between the shield structure and the shield tube may favourably be avoided.

In a preferred embodiment, the shield tube is configured to reduce a heat load on the at least one solenoid coil of the main magnet.

Even though the shield tube preferably comprises or consists of a material having an intermediate electrical conductivity, the shield tube may still allow for a generation of eddy currents (or screening currents) along an electrically conducting surface of the shield tube in dependence of a primary (electro-)magnetic field, particularly a stray field of a gradient magnetic field. The screening currents generated in the shield tube may favourably substitute eddy currents generated in conducting surfaces of the main magnet and/or the cryogen vessel and thus reduce a cooling demand at a temperature level close to the superconducting temperature.

According to an embodiment, the shield tube has a wall thickness of less than 5 mm, less than 4 mm, or less than 3 mm. The wall thickness of the shield tube may exceed 1 mm. Preferably, the shield tube has a wall thickness of approximately 2 mm.

The shield tube may be configured to act as a secondary shield, screening the main magnet and/or the cryogen vessel from (electro-)magnetic fields, particularly stray fields of a gradient magnetic fields, but also from thermal radiation. Thus, heat loads on the main magnet and/or the cryogen vessel may be reduced, which favourably decreases cooling requirements at very low temperature levels (i. e. temperature levels close to the superconducting temperature) where an efficiency of cooling is particularly low. Consequently, an energy efficiency of a magnetic resonance device comprising an inventive shielding arrangement may favourably be increased in comparison to conventional magnetic resonance devices.

In providing a shield tube consisting of a material having an intermediate electrical conductivity, a screening current in a conducting surface of the shield tube may be lower or scaled down in comparison to materials with higher electrical conductivity. Thus, secondary magnetic fields reaching or interacting with the main magnet and/or a main magnetic field within an imaging volume may be reduced or attenuated. As a consequence, imaging artefacts due to secondary magnetic fields in the imaging volume may favourably be reduced or avoided.

According to an embodiment, the inventive shield structure comprises a plurality of shield tubes. The plurality of shield tubes may be mechanically supported via the shield structure. It is also conceivable that a first shield tube of the plurality of shield tubes provides mechanical support to a second shield tube of the plurality of shield tubes. For example, a second shield tube having a second diameter may be mechanically supported via a first shield tube having a first diameter smaller than the second diameter. Preferably, multiple shield tubes are used in shielding arrangements comprising magnets with high magnetic field strengths, for example magnetic field strengths in excess of 3 T.

A shielding arrangement according to the invention may favourably simplify a construction and/or reduce manufacturing costs in comparison to shielding arrangements comprising a plurality of enclosing vessels as shielding structures. For example, the shield tube may take up less radial space than a conventional cryogen vessel or similar enclosing vessel and may also be easier to manufacture and/or to install within the shielding arrangement. Furthermore, a shield tube according to an embodiment described above may have an insignificant effect on a mechanical resonance behaviour of the shield structure. Thus, the shield tube may favourably be installed in shielding arrangements for different magnets without requiring a modification of the shield structure.

The inventive shielding arrangement favourably allows for an optimization or tuning of a provided shielding effect in relation to an occurrence of undesirable secondary magnetic fields.

As a further advantage, the inventive shielding arrangement allows for high performance gradient systems to be used without increasing a cryogen boil-off or a risk of quenching of the main magnet.

According to an embodiment, the inventive shielding arrangement comprises a flexible thermal conductor thermally connected to the shield tube and the shield structure.

A flexible thermal conductor may be plastically or elastically deformable. Particularly, the flexible thermal conductor may be manually deformable. In a preferred embodiment, the flexible thermal conductor comprises a flexible metal fabric, such as a metal braid, a metal mesh, or a metal weave. The flexible metal fabric may comprise or consist of a metal with a high thermal conductivity, such as copper, gold, aluminium, platinum, silver, or the like. However, it is also conceivable that the flexible thermal conductor comprises a flexible carbon fabric or a fabric comprising other highly conductive materials. In a preferred embodiment, the flexible thermal conductor consists of a copper braid.

A flexible thermal conductor may allow for a simple and/or cost-effective implementation of a thermal contact between the shield tube and the shield structure.

According to an embodiment of the inventive shielding arrangement, the at least one spacer occupies an entire volume between the inner wall of the shield structure and the shield tube.

The at least one spacer may fill a gap or a free volume between the shield structure and the shield tube. For example, a shape of the at least one spacer may correspond to a shape of the gap or free volume between the shield structure and the shield tube. However, the shielding arrangement may also comprise a plurality of spacers which, taken together, fill the gap or free volume between the shield structure and the shield tube.

In a preferred embodiment, a shape of the at least one spacer or the plurality of spacers corresponds to a hollow cylinder. In providing at least one spacer occupying an entire volume between the shield structure and the shield tube, the shield tube may be mechanically supported over an entire axial dimension of the shield tube. Thus, a relative movement between the shield structure and the shield tube may favourably be reduced or avoided. Furthermore, a transport of mechanical energy from the shield tube to the shield structure may favourably be improved.

In a preferred embodiment, the inventive shielding arrangement comprises at least two spacers spaced apart along an axial direction defined by the at least one solenoid coil of the main magnet to provide a gap between the inner wall of the shield structure and the shield tube.

The shielding arrangement may comprise at least two spacers. A first spacer may be located at a first end section of the shield tube and a second spacer may be located at a second end section of the shield tube. However, the shield tube may be supported by additional spacers located in proximity to a middle section of the shield tube or a symmetry plane of the shield tube oriented perpendicular to a cylinder axis.

The at least two spacers are spaced apart along an axial direction defined by the at least one solenoid coil of the main magnet. For example, a first spacer may be spaced or separated from a second spacer along the axial direction of the main magnet. However, the first spacer and the second spacer may also be located at different angles of rotation with respect to the cylinder axis of the main magnet. For example, the first spacer and the second spacer may be located at different positions along a circumference of the shield tube.

The gap between the at least two spacers may impact a mechanical coupling between the shield structure and the shield tube. The inventive shielding arrangement may favourably allow for tuning of a transfer of mechanical energy between the shield structure and the shield tube in dependence of a dimension of the gap and/or dimensions of the at least two spacers.

According to a preferred embodiment of the inventive shielding arrangement, the at least one spacer and/or the shield tube comprise a plurality of layers.

The plurality of layers may be arranged between the inner wall of the shield structure and the shield tube. However, at least one layer of the shield tube may be arranged on a surface of the shield facing away from the inner wall of the shield structure.

The plurality of layers may comprise or consist of two or more layers, for example a first layer and a second layer. A material composition of the first layer may differ from a material composition of the second layer. The at least one spacer and/or the shield tube may comprise one or more further layers having a material composition which is different from the material composition of the first layer and/or the material composition of the second layer. For example, a layer of the plurality of layers may consist of a metal, a metal coating, a glass-reinforced plastic (GRP), an epoxy resin, a damping material, a ceramic, a metal alloy, or a composite material. A suitable metal may be stainless steel, steel, aluminium, copper, or the like. Examples for suitable metal coatings are aluminium, copper, silver, gold, and platinum.

Preferably, the layers of the plurality of layers are stacked in a radial direction of the shielding arrangement. For example, the plurality of layers may form a sandwich structure having multiple layers stacked on top of each other in the radial direction of the shield tube. It is also conceivable, however, that the plurality of layers of the at least one spacer and/or the shield tube is distributed along an axial direction of the shield tube. Thus, a mechanical support of the shield tube, but also a mechanical coupling between the shield tube and the shield structure, may be tuned locally, for example at specific locations along an axial direction of the shield tube.

In providing at least one spacer and/or a shield tube comprising a plurality of layers, a mechanical coupling between the shield tube and the shield structure, but also a mechanical resonance behaviour of the shield structure, may favourably be tuned without requiring modifications to a design of the shield structure.

In a preferred embodiment of the inventive shielding arrangement, at least one layer of the plurality of layers consists of a metal coating.

The metal coating may consist of any of the metals mentioned above. Preferably, the metal coating consists of a metal having a high electrical conductivity, such as copper, aluminium, gold, silver, or platinum. A thickness of the metal coating may be less than 1.5 mm, less than 1 mm, less than 0.5 mm, or less an 0.25 mm.

In a preferred embodiment, at least one surface of the shield tube comprises a metal coating. It is also conceivable, however, that at least one layer of the at least one spacer consists of a metal coating.

In providing a shield tube and/or at least one spacer comprising a metal coating, a shielding effect of the shielding arrangement may favourably be improved. In particular, the metal coating may facilitate the generation of screening currents, thus decreasing heat loads on the main magnet and/or the cryogen vessel as well as a cooling demand at a temperature level close to the superconducting temperature. Furthermore, the thickness of the metal coating may favourably be tuned to limit the time-constant of eddy currents induced in the metal coating.

According to an embodiment of the inventive shielding arrangement, the at least one spacer and/or the shield tube comprise a damping element. Alternatively or additionally, the at least one spacer is arranged in such a way to reduce a transfer of mechanical energy between the shield structure and the shield tube.

Reducing a transfer of mechanical energy between the shield structure and the shield tube may comprise reducing or alleviating a mechanical coupling between the shield structure and the shield tube.

The at least one spacer may be configured to limit an area or a surface in contact with the shield tube. For example, a radially outer section of the at least one spacer in contact with the shield tube may comprise a ridge thinning towards the shield tube. It is also conceivable that the at least one spacer comprises one or more pins, knobs, or buttons mechanically supporting the shield tube at discrete locations along an outer surface or circumference of the shield tube. Furthermore, at least two spacers may be spaced in such a way that one or more gaps are formed between the at least two spacers. The one or more gaps may represent sections, where the shield tube can vibrate without transferring mechanical vibrations, particularly undesirable mechanical oscillations, to the shield structure.

According to an embodiment, the at least one spacer and/or the shield tube may comprise a damping element configured to absorb or dissipate mechanical energy. For example, the at least one spacer may comprise a damping material, multiple layers of a damping material, an elastic element, or a plurality of elastic elements configured to absorb mechanical vibrations. In one embodiment, the at least one spacer may comprise a spring and/or an elastic material (e. g. an elastomer and/or an elastic foam material) configured to transform mechanical vibrations into an elastic deformation and/or heat energy. Any heat energy released by the elastic deformation of the at least one spacer may favourably be transferred to a cold source connected to the shield structure at a temperature level well above the superconducting temperature.

It is conceivable that the shield tube comprises a damping element according to an embodiment described above.

In providing a shield tube and/or at least one spacer configured to reduce a transfer of mechanical energy between the shield tube and the shield structure, an effect of the shield tube on the mechanical resonance behaviour of the shield structure may favourably be reduced or avoided.

According to an embodiment of the inventive shielding arrangement, the at least one spacer and/or the shield tube comprise a rigid material. Alternatively or additionally, the at least one spacer is arranged in such a way to improve a transfer of mechanical energy between the shield structure and the shield tube.

The at least one spacer and/or the shield tube may be configured to transfer mechanical vibrations from the shield tube to the shield structure. The at least one spacer may consist of a rigid or inelastic material, such as steel, stainless steel, or glass-reinforced plastic. The rigid material of the at least one spacer may be in mechanical contact with the shield tube and the shield structure. The shield tube may consist of a rigid material such as steel or stainless steel. In a preferred embodiment, a layer of the at least one spacer and/or a layer of the shield tube consist of a rigid material.

The at least one spacer may be arranged in such a way to improve a transfer of mechanical energy between the shield structure and the shield tube. According to an embodiment, a large share of an outer surface of the shield tube may be in contact with the at least one spacer. For example, more than 50 %, more than 60 %, more than 70 % or more than 80 % of the outer surface or circumference of the shield tube may be in contact with the at least one spacer.

In a preferred embodiment, the at least one spacer is configured to transfer mechanical vibrations of selected mechanical frequencies and/or selected mechanical oscillations from the shield tube to the shield structure. For example, the at least one spacer may be located at one or more selected nodes along an outer circumference of the shield tube. The one or more selected nodes may be characterized by a particular oscillation frequency and/or oscillation amplitude of a section of a wall of the shield tube caused by a mechanical vibration of the shield tube. In locating the at least one spacer at one or more selected nodes along an outer circumference of the shield tube, selected mechanical vibrations and/or selected mechanical frequencies may favourably be transferred from the shield tube to the shield structure.

The mechanical vibrations of the shield tube may favourably be used for tuning of a mechanical resonance behaviour of the shield structure, e. g. to modify or decrease a peak mechanical resonance of the shield structure. Furthermore, in improving or enhancing a mechanical coupling between the shield tube and the shield structure, undesirable resonance frequencies of the shield tube may favourably be attenuated.

In an embodiment of the inventive shielding arrangement, the shield structure comprises a plurality of layers, wherein at least one layer of the plurality of layers is configured to modify a mechanical resonance behaviour of the shield structure in dependence of a mechanical resonance behaviour of the shield tube.

The inner wall of the shield structure may represent a layer of the shield structure. According to an embodiment, the at least one layer of the shield structure is mechanically coupled with the shield tube via the at least one spacer and/or the inner wall of the shield structure. Preferably, the at least one layer of the shield structure represents an additional layer of material mechanically connected to a surface of the shield structure, e. g. an inner surface and/or an outer surface of the inner wall of the shield structure. A material of the at least one layer of the shield structure may be different from a material of the shield structure. For example, the shield structure may consist of a highly conductive material, such as aluminium, copper, or the like. The at least one layer may consist of a material with a high stiffness, such as steel or stainless steel.

According to an embodiment, the at least one layer of the shield structure may form a part of the at least one spacer mechanically connected to the shield structure. However, the at least one layer of the shield structure may also be mechanically connected to a surface of the inner wall of the shield structure, particularly a surface of the inner wall of the shield structure facing away from the shield tube.

Preferably, the at least one layer of the shield structure is configured to mitigate or compensate for a mechanical resonance behaviour of the shield tube. For example, the at least one layer of the shield structure may be configured in accordance with the at least one spacer configured to improve a transfer of mechanical energy between the shield structure and the shield tube and/or to transfer mechanical vibrations of selected mechanical frequencies and/or selected mechanical oscillations from the shield tube to the shield structure according to an embodiment described above.

The at least one layer of the shield structure may be configured to increase a stiffness of the shield structure. Particularly, the at least one layer of the shield structure may be configured to increase a stiffness of the inner wall or inner tube of the shield structure. The stiffness of the shield structure may be characterized by an extent to which the shield structure resists deformation in response to an applied force, such as a mechanical vibration caused by the shield tube. Preferably, the at least one layer of the shield structure comprises or consists of steel or stainless steel.

A mechanical resonance behaviour of the shield structure and/or the shield tube may be characterized by a resonance frequency of the shield structure and/or the shield tube under a certain operating condition, e. g. a magnetic resonance measurement carried out via a magnetic resonance device comprising an inventive shielding arrangement. In a preferred embodiment, a thickness and/or a material of the at least one layer of the shield structure is chosen in such a way to compensate for one or more peak mechanical resonance frequencies of the shield tube.

In providing a shield structure comprising at least one layer configured to increase a stiffness of the shield structure, mechanical vibrations introduced or caused by the shield tube may favourably be attenuated. Furthermore, increasing the stiffness of the shield structure may favourably allow for a reduction of a wall thickness of the shield structure. Thus, the shield structure may take up less space in a radial direction of the shielding arrangement.

According to an embodiment, the at least one layer of the shield structure comprises a damping material configured to dissipate mechanical energy. For example, the at least one layer of the shield structure may comprise an elastic material according to an embodiment described above.

In providing a shield structure comprising at least one layer configured to modify a mechanical resonance behaviour of the shield structure mechanical vibrations introduced or caused by the shield tube may favourably be balanced or compensated.

According to an embodiment, the inventive shielding arrangement comprises a gradient system including at least one gradient coil configured to generate a gradient magnetic field in a volume circumferentially encompassed by the inner wall of the shield structure, wherein the shield tube is spaced from the gradient coil in such a way to reduce a screening current induced in the shield tube via a stray field of the gradient magnetic field.

The gradient system may comprise one or more gradient coils. A gradient coil may be configured to generate a gradient magnetic field in an imaging region circumferentially encompassed by the shielding arrangement, particular the inner wall of the shield structure and/or the gradient coil. In a preferred embodiment, the gradient system comprises at least a first gradient coil and a second gradient coil. The first gradient coil may be configured to generate a first gradient magnetic field in the imaging region. Likewise, the second gradient coil may be configured to generate a second gradient magnetic field in the imaging region. The first gradient magnetic field may be oriented essentially perpendicular to the second gradient magnetic field. The gradient system may further comprise a third gradient coil configured to generate a third gradient magnetic field within the imaging region. It is conceivable, that the third gradient magnetic field is oriented substantially perpendicular to the first gradient magnetic field and the second gradient magnetic field.

The shield tube may circumferentially encompass the gradient system. In a preferred embodiment, the gradient system is arranged in a tubular or cylindrical form. A diameter of the shield tube exceeds a diameter of the gradient system.

Preferably, the shield tube is positioned relative to the gradient system in such a way to reduce a generation of screening currents along an electrically conducting surface of the shield tube in dependence of the gradient magnetic field. For example, a distance between a point on an inner surface of the shield tube and a point on an outer surface of the gradient system along a surface normal of the inner surface of the shield tube may exceed 1 cm, 2 cm, 4 cm, 6 cm, 8 cm, or even 10 cm. Of course, the distance between the shield tube and the gradient system may depend on a magnetic field strength of a gradient coil as well as on a magnetic field strength of a main magnet.

In spacing the shield tube from the gradient system according to an embodiment described above, mechanical vibrations of the shield tube may favourably be reduced or limited. Furthermore, a reduction of a screening current in a conductive surface of the shield tube may also reduce or attenuate secondary magnetic fields reaching or interacting with the main magnet and/or a main magnetic field within the imaging volume. Thus, imaging artifacts due to secondary magnetic fields in the imaging volume may favourably be reduced or avoided.

According to an embodiment, the inventive shielding arrangement comprises a cryogen vessel enclosing the main magnet, wherein an inner diameter of the cryogen vessel exceeds a diameter of the shield tube and wherein the cryogen vessel circumferentially encompasses the shield tube.

A cryogen vessel may be a fluid-tight container configured to enclose the main magnet and a fluid, particularly a cryogen. At least a part of the main magnet may be immersed in a liquid portion of the fluid within the cryogen vessel. In a preferred embodiment, the cryogen vessel forms a double-walled hollow cylinder enclosing the main magnet between an outer wall and an inner wall of the cryogen vessel. The outer tube and the inner tube may be mechanically connected via annular end walls. A cylinder axis or axis of rotational symmetry of the cryogen vessel may be oriented in parallel to or correspond to an axis of rotational symmetry defined by the solenoid coil of the main magnet.

The cryogen vessel may be configured to provide a shielding effect. Preferably, the cryogen vessel is maintained at a temperature level close to the superconducting temperature of the main magnet. In one embodiment, the cryogen vessel and/or the cryogen contained within the cryogen vessel are thermally connected to a second stage of a cryocooler. Thus, any heat energy absorbed from the cryogen vessel via the cryocooler may have to be absorbed at a very low temperature level with low efficiency.

The shield tube may be arranged within a tubular space or cavity formed by the cryogen vessel, particularly the inner wall of the cryogen vessel. In a preferred embodiment, the shield tube is arranged between the inner wall of the cryogen vessel and the inner wall or inner tube of the shield structure.

A shielding effect of an inventive shielding arrangement according to an embodiment described above may favourably surpass or exceed a shielding effect of a conventional shielding arrangement without a shield tube. In arranging a shield tube between the inner wall of the cryogen vessel and the gradient system, stray fields of the gradient magnetic fields may at least partially be cancelled out by eddy currents generated within conducting surfaces of the shield tube. Thus, mechanical vibrations and/or heat loads associated with eddy currents may at least partially be shifted to the shield tube, which can be cooled at higher temperatures in comparison to the cryogen vessel. As the cooling efficiency of cryocoolers typically increases with increasing temperature levels, the shield tube may favourably allow for a lower cooling demand at a level of the superconducting temperature and a reduced power demand of the cryocooler.

The inventive magnetic resonance device is configured to carry out a magnetic resonance measurement of an object positioned within an imaging region of the magnetic resonance device. The magnetic resonance device comprises a gradient system including at least one gradient coil and a shielding arrangement according to an embodiment described above.

The inventive magnetic resonance device may be configured to acquire magnetic resonance data from the object positioned within the imaging region of the magnetic resonance device. Preferably, the magnetic resonance device is configured to acquire magnetic resonance image data, particularly diagnostic magnetic resonance image data, from the object positioned within the imaging region. The object may be a patient, particularly a human or an animal.

Preferably, the inventive magnetic resonance device is a closed bore scanner. A closed bore scanner may comprise a substantially cylindrical bore circumferentially enclosing the imaging region. The main magnet of the closed bore scanner may comprise one or more solenoid coils circumferentially encompassing the imaging region along an axial direction or an axis of rotational symmetry of the cylindrical bore. The one or more solenoid coils may comprise a wire having negligible electrical resistance at (or below) a superconducting temperature. A direction of the main magnetic field provided via the main magnet may be oriented substantially in parallel to a direction of access of the object to the imaging region and/or the axial direction of the cylindrical bore.

The magnetic resonance device may comprise at least one cryocooler. The at least one cryocooler may be configured to cool components of the magnetic resonance device. For example, the at least one cryocooler may be configured to cool the main magnet, the shield structure, the shield tube, the cryogen vessel, and the like.

The at least one cryocooler may be configured to provide a temperature close to or lower than a superconducting temperature of a superconducting material of the main magnet. For example, the superconducting temperature of the main magnet may be in the range of 3 K to 100 K, preferably in the range of 3 K to 6 K, 5 K to 10 K, 30 K to 60 K, or 60 K to 90 K. The at least one cryocooler may be implemented as a pulse tube refrigerator, a Gifford-McMahon refrigerator, a Stirling cryocooler, a Joule-Thomson cryocooler, or the like.

In a preferred embodiment, the at least one cryocooler is thermally and mechanically connected to the main magnet, the shield structure, and the shield tube. The at least one cryocooler may be configured to maintain the main magnet and the shield structure, but also the shield tube, at different temperature levels.

The at least one cryocooler may comprise a cold head with one or more cooling stages. In case the cold head comprises multiple cooling stages, each cooling stage may have a different temperature level. Alternatively, the inventive magnetic resonance device comprises a first cryocooler configured for cooling the main magnet, and a second cryocooler configured for cooling the shield structure and/or the shield tube. Preferably, a temperature level provided by the second cryocooler exceeds a temperature level provided by the first cryocooler.

According to an embodiment, the at least one cryocooler comprises at least a first cooling stage and a second cooling stage. The first cooling stage is thermally and mechanically connected to the shield structure and the shield tube. The second stage is thermally and mechanically connected to the main magnet. Preferably, a temperature level of the first cooling stage exceeds a temperature level of the second stage. For example, the first cooling stage may be configured to provide a temperature in the range between 40 K to 180 K, preferably in the range between 40 K to 60 K, 60 K to 100 K, or 100 K to 180 K. The second cooling stage may be configured to provide a temperature level close to the superconducting temperature of the main magnet.

The inventive magnetic resonance device may be configured as a "dry" system. A "dry" system may comprise a minimum amount of cryogen or no cryogen at all. For example, the inventive magnetic resonance system may comprise one or more small cryogen vessels thermally connected to the main magnet via a solid thermal conductor. The small cryogen vessels may contain a volume of less than 10 l, less than 5 l, or less than 1 1 of cryogen. In an embodiment, a cryogen vessel is omitted. Thus, the main magnet may be cooled entirely via thermal conduction.

In an alternative embodiment, the inventive magnetic resonance device is configured as a "wet" system. A "wet" system may comprise a cryogen vessel containing a fluid or a cryogen with a low boiling point like Argon, Nitrogen, Neon, Helium, or the like. The predefined temperature level may substantially correspond to a superconducting temperature of the main magnet.

It is conceivable that components of the magnetic resonance device, such as the main magnet, the cryogen vessel, the shield structure, the shield tube, and the like, are thermally connected to the cryocooler. Preferably, the components of the magnetic resonance device are thermally connected to the cryocooler via a solid thermal conductor, a convection loop, and/or a heat pipe.

The inventive magnetic resonance device shares the advantages of the inventive shielding arrangement.

Further advantages and details of the present invention may be recognized from the embodiments described below as well as the drawings. The figures show:
Fig. 1 a schematic representation of an embodiment of an inventive magnetic resonance device,
Fig. 2 a schematic representation of an embodiment of an inventive magnetic resonance device,
Fig. 3 a schematic representation of an embodiment of an inventive shielding arrangement,
Fig. 4 a schematic representation of an embodiment of an inventive shielding arrangement,
Fig. 5 a schematic representation of a section of an embodiment of an inventive shielding arrangement,
Fig. 6 a schematic representation of a shield tube and a spacer of an inventive shielding arrangement,
Fig. 7 a schematic representation of a section of an embodiment of an inventive shielding arrangement,
Fig. 8 a schematic representation of an embodiment of an inventive shielding arrangement,
Fig. 9 a schematic representation of an embodiment of an inventive shielding arrangement,
Fig. 10 a comparison of mechanical resonance behaviours of different embodiments of shielding arrangements.

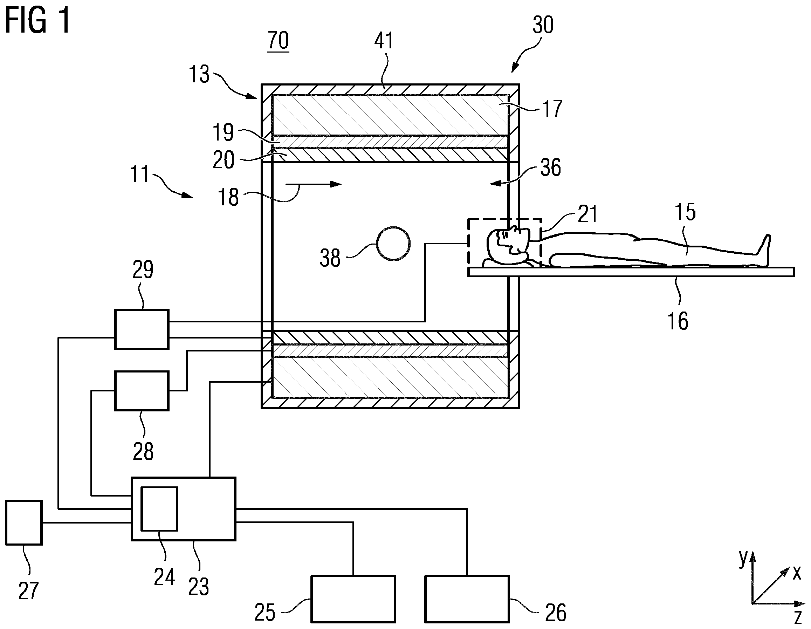

Fig. 1 shows an embodiment of a magnetic resonance device 11 according to the invention. In the illustrated example, the magnetic resonance device 11 comprises a static field magnet 17 or main magnet configured to provide a homogenous, static magnetic field 18 (B0 field) comprising an imaging volume 38. The static magnetic field 18 permeates an imaging region 36 configured for receiving an imaging object, such as a patient 15. The imaging region 36 may correspond to a patient bore configured for accommodating a patient during a magnetic resonance measurement. The imaging region 36 is encompassed by the shielding arrangement 30 in a circumferential direction.

In the depicted example, the magnetic resonance device 11 comprises a patient support 16 configured to transport the patient 15 into the imaging region 36. Particularly, the patient support 16 is configured to transport a diagnostically relevant body region of the patient 15 into the imaging volume 38 or isocentre of the magnetic resonance device 11. Typically, the shielding arrangement 30 of the magnetic resonance device 11 is concealed in a housing 41.

The magnetic resonance device 11 may comprise a gradient system 19 configured to provide gradient magnetic fields used for spatial encoding of magnetic resonance signals acquired during a magnetic resonance measurement. The gradient system 19 is activated or controlled by a gradient controller 28 via an appropriate current signal. It is conceivable that the gradient system 19 comprises one or more gradient coils configured to generate gradient magnetic fields in different, preferably orthogonally oriented, spatial directions.

The magnetic resonance device 11 may comprise an integrated radiofrequency antenna 20 (i. e. a body coil). The radiofrequency antenna 20 may be operated via a radiofrequency controller 29 that controls the radiofrequency antenna 20 to generate a high frequency magnetic field and emit radiofrequency excitation pulses into the imaging region 36. The magnetic resonance device 11 may further comprise a local coil 21. The local coil 21 may be positioned on or in proximity to the diagnostically relevant region of the patient 15. The local coil 21 may be configured to emit radiofrequency excitation pulses into the patient 15 and/or receive magnetic resonance signals from the patient 15. It is conceivable, that the local coil 21 is controlled via the radiofrequency controller 29.

Preferably, the magnetic resonance device 11 comprises a control unit 23 configured for controlling the magnetic resonance device 11. The control unit 23 may comprise a processing unit 24 configured to process magnetic resonance signals and reconstruct magnetic resonance images. The processing unit 24 may also be configured to process input from a user of the magnetic resonance device 11 and/or provide an output to a user. For this purpose, the processing unit 24 and/or the control unit 23 can be connected to a display unit 25 and an input unit 26 via a suitable signal connection. For a preparation of a magnetic resonance measurement, preparatory information, such as imaging parameters or patient information, can be provided to the user via the display unit 25. The input unit 26 may be configured to receive information and/or imaging parameters from the user.

Of course, the magnetic resonance device 11 may comprise further components that magnetic resonance devices usually offer. The general operation of a magnetic resonance device 11 is known to those skilled in the art, so a more detailed description is omitted.

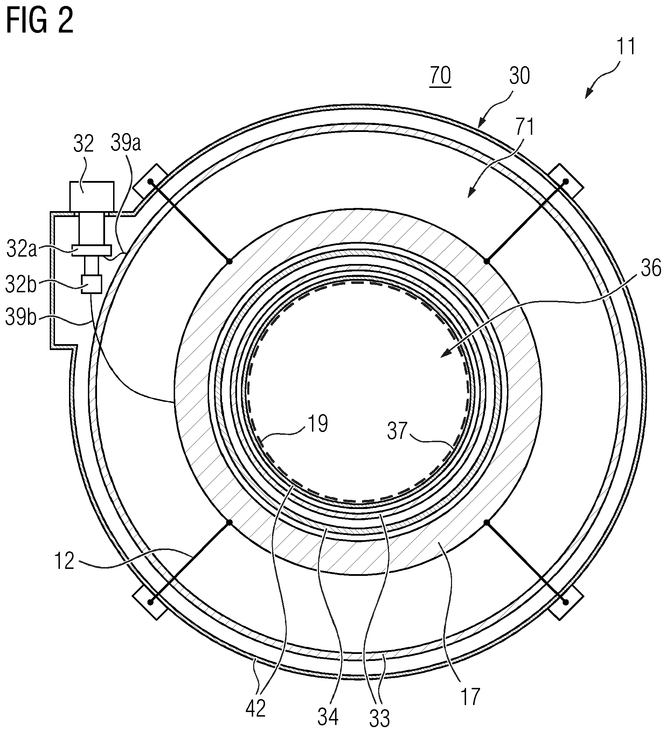

Fig. 2 shows a sectional view of the inventive magnetic resonance device 11 according to Fig. 1. In the depicted example, the shielding arrangement 30 comprises an outer vacuum chamber 42 providing an outer enclosure for the components of the shielding arrangement 30. The outer vacuum chamber 42 separates a surrounding environment 70 from a vacuum region 71 encompassed by the outer vacuum chamber 42. In a preferred embodiment, the outer vacuum chamber 42 forms a double-walled hollow cylinder comprising an outer shell and an inner shell. The components of the shielding arrangement 30 may be housed within or enclosed by the outer shell and the inner shell of the outer vacuum chamber 42. The inner shell of the outer vacuum chamber 42 may form the patient bore 37 encompassing the imaging region 36 in a circumferential direction.

In the illustrated embodiment, the shielding arrangement 30 comprises a shield structure 33 and a shield tube 34. The shield structure 33 is embodied as double-walled hollow cylinder, whereas the shield tube 34 is embodied as a tube or a hollow cylinder. The shield tube 34 and the main magnet 17 are enclosed between an outer wall and an inner wall of the shield structure 33. Thus, the main magnet 17 is confined by the outer wall of the shield structure 33 and the shield tube 34.

Preferably, the shielding arrangement 30 comprises one or more suspension rods 12 mechanically connected to the outer shell of the outer vacuum chamber 42 and the main magnet 17. The suspension rods 12 may extend through passages or holes in the shield structure 33 to provide a mechanical connection between the outer shell of the outer vacuum chamber 42 and the main magnet 17.

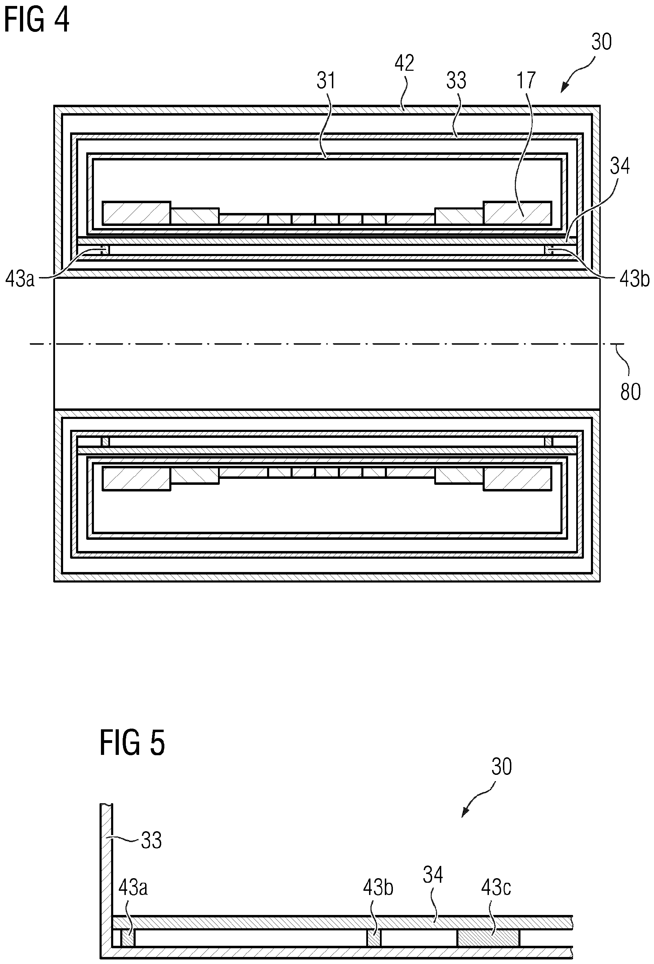

The magnetic resonance device 11 further comprises a cryocooler 32 mounted on the outer vacuum chamber 42. The cryocooler 32 may be configured to cool the main magnet 17, the shield structure 33, the shield tube 34, but also other components of the shielding arrangement 30, such as a cryogen vessel 31 (see Fig. 4).

The cryocooler 32 typically comprises a compressor supplying pressurized gas to the cryocooler 32 (not shown). According to the embodiment shown in Fig. 2, the cryocooler 32 includes a cold head comprising one or more cooling stages 32a and 32b. Preferably, a first cooling stage 32a of the cold head is thermally connected to the shield structure 33, whereas a second cooling stage 32b of the cold head is thermally connected to the main magnet 17. In a preferred embodiment, the first cooling stage 32a provides a temperature level of about 50 K, whereas the second cooling stage 32b provides a temperature level of about 4 K. In "dry" systems, the cooling stages 32a and 32b of the cryocooler 32 can be thermally connected to components of the shielding arrangement 30 via solid thermal conductors 39a and 39b. It is also conceivable that the magnetic resonance device 11 comprises one or more small cryogen vessels (not shown) thermally connected to the cryocooler 32 via a solid thermal conductor, a heat pipe, and/or a convective loop. The one or more cryogen vessels may be thermally connected to the main magnet 17 via a heat exchanger and/or a solid thermal conductor 39.

According to an embodiment, the shield tube 34 is thermally connected to the shield structure 33 via a solid thermal conductor, for example a copper braid (not shown). However, the shield tube 34 may also be thermally connected to the first cooling stage 32a of the cryocooler 32 via a solid thermal conductor 39.

During operation of the magnetic resonance device 11, the shield structure 33 may be maintained at an intermediate temperature, e. g. between 40 K and 60 K, preferably about 50 K. The shield structure 33 may comprise an electrically conductive material configured for shielding the main magnet 17 from thermal radiation, but also from stray fields of the gradient magnetic fields generated via the gradient system 19.

In an alternative embodiment, the shield structure 33 and the main magnet 17 are thermally connected to the same stage of a cryocooler 32. It is also conceivable, that the shield structure 33 and the main magnet 17 are connected to different or separate cryocoolers 32.

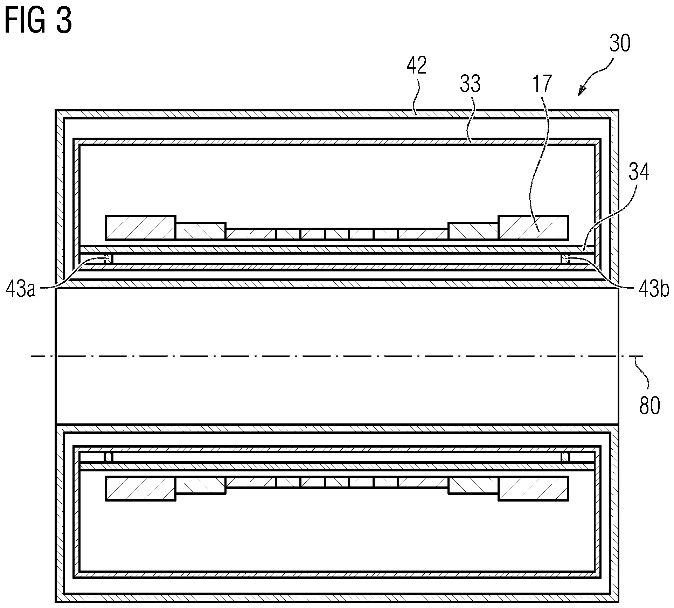

Fig. 3 shows a sectional view of an embodiment of an inventive shielding arrangement 30. In the illustrated example, the shielding arrangement 30 comprises an outer vacuum chamber 42, a main magnet 17, a shield structure 33 and a shield tube 34. The shield tube 34 is mechanically supported via spacers 43a and 43b carried by the shield structure 33. The spacers 43a and 43b are spaced along an axial direction of the shielding arrangement 30. For example, the spacer 43a is spaced from the spacer 43b in an axial direction oriented in parallel to the axis of rotational symmetry 80 of the shielding arrangement 30. In the depicted embodiment, the spacers 43a and 43b are implemented as rings or hollow cylinders circumferentially encompassing the inner wall of the shield structure 33.

The shielding arrangement 30 illustrated in Fig. 3 may be used in "dry" magnetic resonance devices relying mainly on cooling via thermal conduction. However, "dry" magnetic resonance devices may still comprise a small cryogen vessel acting as a thermal buffer. Such a cryogen vessel may be thermally connected to the main magnet 17 via a solid thermal conductor.

Fig. 4 shows a sectional view of a further embodiment of an inventive shielding arrangement 30. In the depicted example, the shielding arrangement 30 comprises a cryogen vessel 31 enclosing the main magnet 17. The cryogen vessel 31 forms a fluid-tight vessel containing a cryogen (not shown). Parts of the main magnet 17 may be immersed within a liquid portion of the cryogen. The cryogen in the cryogen vessel 31 may be in direct contact with the second cooling stage 32b of the cryocooler 32. However, a wall of the cryogen vessel 31 may also be thermally connected to the second cooling stage 32b of the cryocooler via a solid thermal conductor and/or a heat pipe. The cryogen vessel 31, the shield tube 34 and the shield structure 33 of the shielding arrangement 30 may be configured to contribute to a shielding effect of the shielding arrangement 30. The shielding arrangement 30 depicted in Fig. 4 is preferably used in "wet" magnetic resonance devices.

Fig. 5 shows a section of an embodiment of an inventive shielding arrangement 30 comprising a plurality of spacers 43. In the illustrated example, the spacer 43a is arranged at an end section, particularly an axial end, of the shield tube 34. The spacer 43c is arranged at a middle section of the shield tube 34. The spacer 43a may be configured to provide support to the end section of the shield tube 34, whereas the spacer 43c may be configured to prevent sagging of the middle section of the shield tube 34. The spacer 43b, but also further spacers 43 (not shown), may be configured to provide additional support to the shield tube 34. One or more spacers 43 may also be arranged in such a way to tune a mechanical resonance behaviour of the shield tube 34 and/or the shield structure 33.

The spacers 43 shown in Figs. 3 to 5 can be implemented as buttons or pins configured to mechanically decouple the shield structure 33 from the shield tube 34. The spacers 43 may be spaced from each other. Preferably, the spacers 43 are bolted or bonded to an end spinning or an annular end wall of the shield structure 33 and/or the end section of the shield tube 34. It is also conceivable, that the spacers 43 are distributed over an outer circumference of the shield tube 34 in a regular or irregular pattern. However, the spacers 43 may also be implemented as rings, ring segments, plates, hollow cylinders and/or segments of hollow cylinders. According to an embodiment, the spacers 43 are arranged at transverse sections of the shielding arrangement 30 comprising solenoid coils of the main magnet 17.

According to an embodiment, the spacers 43 comprise or consist of a glass-reinforced plastic. However, one or more spacers 43 may also comprise or consist of aluminium, steel, stainless steel, epoxy resin, a damping element, and/or a damping material. According to a preferred embodiment, one or more spacers 43 consist of a plurality of layers.

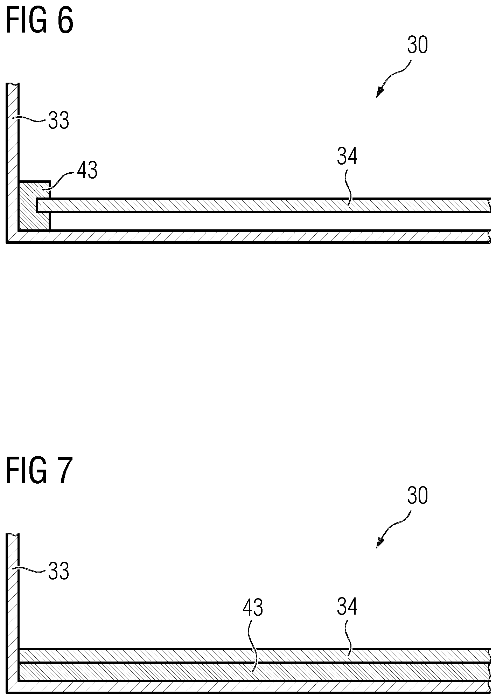

According to the embodiment illustrated in Fig. 6, the spacer 43 comprises a recess or cut-out configured to accommodate a section of the shield tube 34, thus providing a form-locking mechanical connection between the spacer 43 and the shield tube 34.

Fig. 7 shows a further embodiment of an inventive shielding arrangement 30. In the depicted example, the spacer 43 fills an entire gap or a free volume between the shield structure 33 and the shield tube 34. The spacer 43 may consist of a single layer of material, such as glass-reinforced plastic, aluminium, stainless steel, or epoxy resin. However, the spacer 43 may also comprise a plurality of layers made of different materials. For example, the spacer 43 depicted in Fig. 7 may consist of a series of layers stacked in a radial direction of the shielding arrangement 30. According to an embodiment, the shielding arrangement 30 comprises a plurality of spacers 43 which, taken together, fill the entire gap or free volume between the shield structure 33 and the shield tube 34.

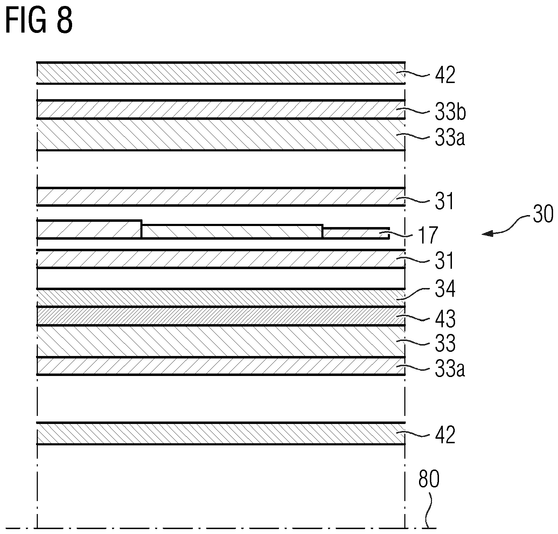

Fig. 8 shows a radial section of an embodiment of the inventive shielding arrangement 30 comprising a cryogen vessel 31. In the depicted example, the shield structure 33 comprises a layer 33a configured to increase a stiffness of the inner wall or inner tube of the shield structure 33. Preferably, the shield structure 33 consists of aluminium, particularly aluminium 5005, whereas the layer 33a of the shield structure 33 consists of stainless steel. The shield structure 33 and the layer 33a may be thermally connected to the first cooling stage 32a of the cryocooler 32 and maintained a temperature level of approximately 50 K.

The shield tube 34 may be supported via one or more spacers 43 according to an embodiment described above. Depending on the location of the radial section along the axis 80, a gap or free volume between the shield tube 34 and the inner wall of the shield structure 33 may be filled by a spacer 43 according to an embodiment described above. However, the gap may also be devoid of any structures or components of the shielding arrangement 30. For example, there may be one or more vacuum regions between the shield tube 34 and the inner wall of the shielding structure 33.

The shield tube 34 may be thermally connected to the shield structure 33 via a separate thermal conductor (not shown) and/or via one or more spacers 43. Thus, the shield tube 34 may be indirectly connected to the cryocooler and exhibit a higher temperature as compared to the shield structure 33. For example, the shield tube 34 may be maintained at a temperature level between 60 K and 70 K during operation of the shielding arrangement 30 in a magnetic resonance device 11. Preferably, the shield tube 34 consists of stainless steel having a thickness of approximately 2 mm.

In the embodiment shown in Fig. 8, the cryogen vessel 31 enclosing the main magnet 17 consists of stainless steel. Preferably, the cryogen vessel 31 is thermally connected to the second cooling stage 32b of the cryocooler 32 and maintained at a temperature of approximately 4 K during operation of the shielding arrangement 30 in a magnetic resonance device 11.

The inner shell and outer shell of the outer vacuum chamber 42 preferably consist of steel, stainless steel, or another resilient metal.

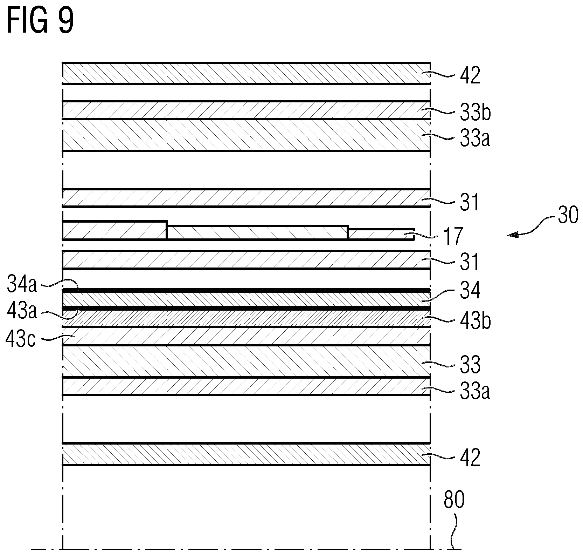

Fig. 9 shows a radial section of a further embodiment of the inventive shielding arrangement 30. In the depicted embodiment, the spacer 43 consists of three layers 43a, 43, and 43c. In contrast to the embodiment depicted in Fig. 8, a layer 43c is mechanically connected to an outer surface of the inner wall of the shield structure 33. The layer 43c may consist of stainless steel and increases the stiffness of the shield structure 33. Preferably, the layer 43c is materially bonded to the inner tube of the shield structure 33, for example via an adhesive such as an epoxy resin.

The spacer 43 further comprises a layer 43b. The layer 43b may comprise a continuous piece or multiple spaced pieces of glass-reinforced plastic. However, the layer 43b may be implemented according to an embodiment described above.

The layer 43a is formed by a metal coating. The metal coating preferably consists of a metal having a high electrical conductivity at 300 K, such as copper, aluminium, gold, silver, or platinum. Preferably, the layer 43a is coated onto an inner surface of the shield tube 34.

According to the embodiment depicted in Fig. 9, an outer surface of the shield tube 34 may comprise a further metal coating 34a. The further metal coating 34a may correspond to a metal coating described above. However, since the metal coating 34a is disposed on the outer surface of the shield tube 34, it does not form a part of the spacer 43.

According to an embodiment of the shielding arrangement 30, one or more layers of the spacer 43 (e. g. layer 43a or layer 43b), the shield tube 34 (e. g. layer 34a), but also the shield structure 33 (e. g. layer 33a) may be omitted.

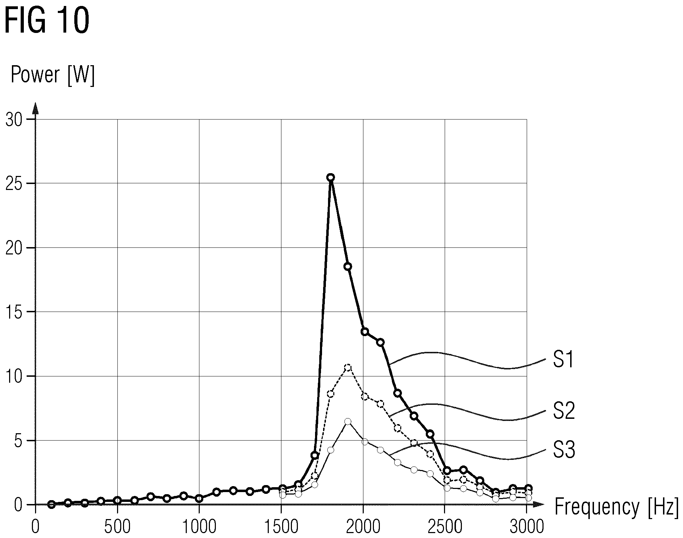

Fig. 10 shows results of a simulation of the mechanical resonance behaviour of the shield structure 33. On the X-axis of the depicted diagram, the frequency of the mechanical vibration is plotted. On the Y-axis, the power of the mechanical vibration is plotted. The plot S1 represents a conventional shielding arrangement without a shield tube 34. The simulated mechanical frequency behaviour shows that the shield structure 33 exhibits a significant resonance frequency at approximately 1800 Hz. A simulation S2 of the inventive shielding arrangement 30 suggests that an addition of an inventive shield tube 34 attenuates the main peak at 1800 Hz without introducing new peaks in the mechanical resonance behaviour of the shield structure 33.

A further simulation S3 of an inventive shielding arrangement 30 suggests, that the peak of the power deposition may even be reduced further by adding a layer 33a configured for stiffening an inner tube of the shield structure 33.

The embodiments described herein are to be recognized as examples. It is to be understood that individual embodiments may be extended by or combined with features of other embodiments if not stated otherwise. The embodiments depicted in the Figs. 1 to 9 are representations that do not necessarily have to be to scale.

## Claims

1. Shielding arrangement (30) for a magnetic resonance device (11), comprising
• a main magnet (17) including at least one solenoid coil,
• a shield structure (33) formed as a double-walled hollow cylinder comprising an outer wall and an inner wall, and
• a shield tube (34) enclosed between the at least one solenoid coil of the main magnet (17) and the inner wall of the shield structure (33),
wherein the main magnet (17) is arranged between the outer wall and the inner wall of the shield structure (33) and wherein the shield tube (34) is mechanically supported by the shield structure (33).

2. Shielding arrangement (30) according to claim 1, wherein the shield tube (34) comprises a material having a thermal conductivity of less than 120 *W*/(*m·K*) at a temperature of 300 K.

3. Shielding arrangement (30) according to claim 1 or 2, comprising a flexible thermal conductor thermally connected to the shield tube (34) and the shield structure (33).

4. Shielding arrangement (30) according to one of the preceding claims, comprising at least one spacer (43) mechanically connected to the shield structure (33) and the shield tube (34), wherein the at least one spacer (43) is configured to maintain a predefined distance between the inner wall of the shield structure (33) and the shield tube (34).

5. Shielding arrangement (30) according to claim 4, wherein the at least one spacer (43) occupies an entire volume between the inner wall of the shield structure (33) and the shield tube (34).

6. Shielding arrangement (30) according to claim 4, comprising at least two spacers (43) spaced apart along an axial direction defined by the at least one solenoid coil of the main magnet (17) to provide a gap between the inner wall of the shield structure (33) and the shield tube (34).

7. Shielding arrangement (30) according to one of the claims 4 or 6, wherein the at least one spacer (43) and/or the shield tube (34) comprise a plurality of layers.

8. Shielding arrangement (30) according to claim 7, wherein at least one layer of the plurality of layers consists of a metal coating.

9. Shielding arrangement (30) according to one of the claims 4 to 8, wherein the at least one spacer (43) and/or the shield tube (34) comprise a damping element and/or wherein the at least one spacer (43) is arranged in such a way to reduce a transfer of mechanical energy between the shield structure (33) and the shield tube (34).

10. Shielding arrangement (30) according to one of the claims 4 to 8, wherein the at least one spacer (43) and/or the shield tube (34) comprise a rigid material and/or wherein the at least one spacer (43) is arranged in such a way to improve a transfer of mechanical energy between the shield structure (33) and the shield tube (34).

11. Shielding arrangement (30) according to one of the claims 4 to 10, wherein the shield structure (33) comprises a plurality of layers and wherein at least one layer of the plurality of layers is configured to modify a mechanical resonance behaviour of the shield structure (33) in dependence of a mechanical resonance behaviour of the shield tube (34).

12. Shielding arrangement (30) according to claim 11, wherein the at least one layer of the shield structure (33) is configured to increase a stiffness of the shield structure (33).

13. Shielding arrangement (30) according to one of the preceding claims, comprising a gradient system (19) including at least one gradient coil configured to generate a gradient magnetic field in a volume circumferentially encompassed by the inner wall of the shield structure (33), wherein the shield tube (34) is spaced from the gradient coil in such a way to reduce a screening current induced in the shield tube (34) via a stray field of the gradient magnetic field.

14. Shielding arrangement (30) according to one of the previous claims, comprising a cryogen vessel (31) enclosing the main magnet (17), wherein an inner diameter of the cryogen vessel (31) exceeds a diameter of the shield tube (34) and wherein the cryogen vessel (31) circumferentially encompasses the shield tube (34).

15. Magnetic resonance device (11) configured to carry out a magnetic resonance measurement of an object positioned within an imaging region (36) of the magnetic resonance device (11), comprising a gradient system (19) including at least one gradient coil and a shielding arrangement (30) according to one of the preceding claims.
